(19) Europäisches Patentamt
European Patent Office
Office européen des brevets

(11) Veröffentlichungsnummer: **0 021 377**
**A1**

(12) **EUROPÄISCHE PATENTANMELDUNG**

(21) Anmeldenummer: **80103490.1**

(22) Anmeldetag: **21.06.80**

(51) Int. Cl.³: **C 07 D 249/08**, C 07 D 403/06, C 07 D 405/06, C 07 D 407/06, A 01 N 43/64

(30) Priorität: **27.06.79 DE 2925896**

(43) Veröffentlichungstag der Anmeldung: **07.01.81 Patentblatt 81/1**

(84) Benannte Vertragsstaaten: **AT BE CH DE FR GB IT LI NL SE**

(71) Anmelder: **HOECHST AKTIENGESELLSCHAFT, Zentrale Patentabteilung Postfach 80 03 20, D-6230 Frankfurt/Main 80 (DE)**

(72) Erfinder: **Mildenberger, Hilmar, Dr., Fasanenstrasse 24, D-6233 Kelkheim (Taunus) (DE)**
Erfinder: **Maier, Thomas, Dr., Rauenthaler Weg 22, D-6000 Frankfurt am Main 71 (DE)**
Erfinder: **Sachse, Burkhard, Dr., An der Ziegelei 30, D-6233 Kelkheim (Taunus) (DE)**

(54) **Derivate des 1,2,4-Triazols, Verfahren zu ihrer Herstellung und ihre Verwendung als Schädlingsbekämpfungsmittel.**

(57) Gegenstand der Erfindung sind neue 1, 2, 4-Triazolderivate der Formel I,

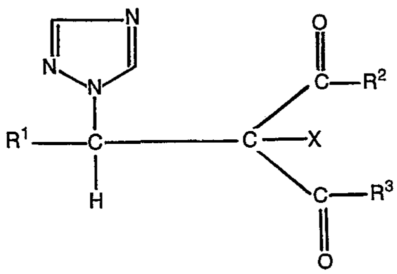

(I)

in der

R¹ → $R^1$ $(C_1-C_{12})$-Alkyl, Cycloalkyl, Cycloalkenyl, gegebenenfalls substituiertes Phenyl, Furanyl, Thienyl oder Pyridyl, und

$R^2$, $R^3$ $(C_1-C_{12})$-Alkyl, Cycloalkyl, gegebenenfalls substituiertes Phenyl, $(C_1-C_{12})$-Alkoxy, $(C_5-C_6)$-Cycloalkoxy oder Benzyloxy, und

X Chlor oder Brom bedeuten,

ferner ein Verfahren zu deren Herstellung sowie deren Verwendung als Schädlingsbekämpfungsmittel.

EP 0 021 377 A1

0021377

HOECHST AKTIENGESELLSCHAFT HOE 79/F 158 Dr.GM/St

# Derivate des 1,2,4-Triazols, Verfahren zu ihrer Herstellung und ihre Verwendung als Schädlingsbekämpfungsmittel

Gegenstand der vorliegenden Erfindung sind neue 1,2,4-Triazolderivate, ihre Herstellung und Verwendung als Schädlingsbekämpfungsmittel insbesondere im Pflanzenschutz.

Die erfindungsgemäßen Verbindungen besitzen die allgemeine Formel I,

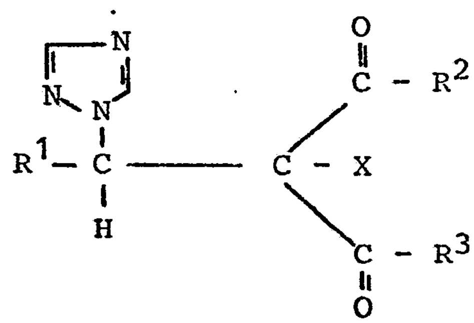

(I)

worin

$R^1$ $(C_1-C_{12})$-Alkyl, Cycloalkyl mit vorzugsweise 5 oder 6 C-Atomen, Cycloalkenyl mit vorzugsweise 5 oder 6 C-Atomen, Phenyl, substituiertes Phenyl, insbesondere ein- bis dreifach durch Alkyl, vorzugsweise $(C_1-C_{12})$-Alkyl, Halogen, $(C_1-C_5)$-Alkoxy, Hydroxy, Nitro oder Dialkylamino mit vorzugsweise $(C_1-C_6)$-Alkylgruppen substituiertes Phenyl, weiterhin Furanyl, Thienyl oder Pyridyl, und

$R^2,R^3$ $(C_1-C_{12})$-Alkyl, Cycloalkyl mit vorzugsweise 5 oder 6 C-Atomen, Phenyl, substituiertes Phenyl, insbesondere ein- bis dreifach durch Alkyl, vorzugsweise $(C_1-C_{12})$-Alkyl, Halogen, $(C_1-C_6)$-Alkoxy oder Hydroxy substituiertes Phenyl, oder $(C_1-C_{12})$-Alkoxy, $(C_5-C_6)$Cycloalkoxy oder Benzyloxy, und

X Chlor oder Brom, vorzugsweise Chlor bedeuten.

Die in den Resten $R^1$, $R^2$, $R^3$ aufgeführten Alkylreste können sowohl geradkettig als auch verzweigt sein. Halogen steht vorzugsweise für Fluor, Chlor, Brom.

Die Verbindungen der allgemeinen Formel I erhält man, indem man Verbindungen der allgemeinen Formel II,

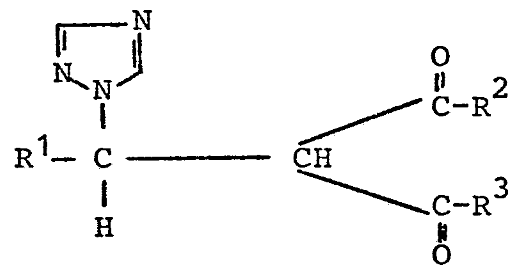

(II)

worin $R^1$, $R^2$, $R^3$ die Bedeutungen wie in Formel I haben, mit einem Halogenierungsmittel umsetzt.

Die Umsetzung erfolgt vorzugsweise in Gegenwart eines Lösungsmittels.
Die Verbindungen der Formel II können z.B., wie in der Patentanmeldung P 28 36 945.1 bereits vorgeschlagen, aus Verbindungen der allgemeinen Formel III,

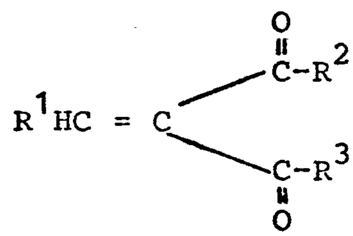

(III)

worin $R^1$, $R^2$ und $R^3$ die Bedeutungen wie in Formel II haben, durch Umsetzung mit 1,2,4-Triazol erhalten werden.

Die Verbindungen der Formel III sind bereits bekannt und lassen sich z.B. durch Knoevenagel-Kondensation darstellen.

Die Halogenierung der Verbindungen der Formel II unter Substitution des Wasserstoffs an dem tertiären C-Atom des ß-Dicarbonylrestes erfolgt in Analogie zu bekannten Verfahren (vgl. z.B. Houben-Weyl, Methoden der Organischen Chemie, Bd. 5/3, 4. Aufl., 1962, Seite 796 und ff.) durch Chlorierung oder Bromierung von Verbindungen der Formel II

mit Halogenierungsmitteln, vorzugsweise mit N-Halogenaminen, wie z.B. N-Chlorsuccinimid, N-Bromsuccinimid, Hexachlormelamin oder N-Chlorurethan.

Für die Arbeitsweise mit den Halogenierungsmitteln, insbesondere mit N-Chlorsuccinimid, N-Bromsuccinimid oder Hexachlormelamin gilt z.B. folgendes: Das Verhältnis der Verbindungen der Formel II zu dem Halogenierungsmittel kann innerhalb weiter Grenzen variiert werden, beispielsweise von 1:0,5 bis zu 1:5 der Stöchiometrie, wobei bei einem Halogenierungsmittel-Unterschuß z.B. der nicht umgesetzte Anteil des Ausgangsproduktes der Formel II beispielsweise in einem Kreisprozeß der Reaktion wieder zugeführt werden kann. Bevorzugt ist das Verhältnis von 1:1 bis 1:2. Selbstverständlich können auch höhere Überschüsse an Halogenierungsmittel eingesetzt werden, was aber im allgemeinen keine Vorteile bringt. Die Ausbeute an Verbindungen der Formel I liegt bei der für die Halogenierung bevorzugten Verfahrensweise bei etwa 60 bis 100 % der Theorie.

Die Halogenierung der Verbindungen der Formel II zu Verbindungen der Formel I wird vorzugsweise in einem unter den Reaktionsbedingungen inerten Lösungsmittel durchgeführt. Geeignete Lösungsmittel sind z.B. halogenierte Kohlenwasserstoffe, insbesondere perchlorierte Kohlenwasserstoffe, wie z.B. Perchloräthylen oder Tetrachlorkohlenstoff, ferner tertiäre Alkohole, wie z.B. tert. Butanol. Die Lösungsmittelmenge kann von Bruchteilen der Menge des Reaktantengemisches bis zu deren Vielfachem betragen. Bevorzugt sind jedoch die halbe bis zur 6-fachen Gewichtsmenge, bezogen auf das Reaktantengemisch. Auch Mischungen aus verschiedenen Lösungsmitteln können eingesetzt werden.

Die Halogenierung kann in einem Temperaturbereich von etwa 20 bis 200°C durchgeführt werden. Bevorzugt sind

Temperaturen von 40 bis 130°C bzw. die Siedetemperatur des jeweiligen Lösungsmittels oder Lösungsmittelgemisches.

Die Reaktionszeit ist nicht kritisch. Sie kann wenige Minuten bis zu 30 Stunden betragen.

Bevorzugt sind Reaktionszeiten von 30 Min. bis zu 20 Stunden, besonders bevorzugt 4 bis 10 Stunden.

Die Isolierung und Reinigung von erfindungsgemäß hergestellten Verbindungen der Formel I erfolgt nach üblichen Methoden.
Bei der vorzugsweise in einem Lösungsmittel durchgeführten Reaktion gehen im allgemeinen die Ausgangskomponenten unter den Reaktionsbedingungen zunächst in Lösung. Mit fortschreitender Reaktion fällt dann in dem Reaktionsgemisch ein Niederschlag aus, der im wesentlichen aus umgesetztem Halogenierungsmittel besteht, während das gewünschte Reaktionsprodukt der Formel I im allgemeinen in Lösung bleibt und von dem Niederschlag abgetrennt werden kann. Das Lösungsmittel und die flüchtigen Bestandteile der Lösung werden bei Temperaturen von vorzugsweise etwa 20 bis 60°C unter Vakuum abdestilliert, wobei im allgemeinen auch überschüssiges Halogenierungsmittel ausfällt und abgetrennt werden kann. Das verbleibende Produkt der Formel I wird isoliert und gegebenenfalls, z.B. durch Behandeln mit geeigneten Lösungsmitteln oder selektiven Lösungsmittel-Fällungsmittel-Gemischen, wie z.B. Diisopropyläther + Chloroform, oder durch Chromatographie weiter gereinigt.

Die erfindungsgemäßen Produkte der Formel I stellen meist zähe, ölige Flüssigkeiten dar, die in manchen Fällen auch kristallisieren.

Den erfindungsgemäßen Verbindungen ist gemeinsam, daß sie insgesamt thermisch empfindlich sind und beim Erhitzen auf Temperaturen von oberhalb 100°C sich allmählich zu zersetzen

beginnen, was die Möglichkeit ihrer destillativen Reinigung, auch unter Hochvakuum, weitgehend verschließt.

Die beanspruchten Verbindungen der Formel I zeichnen sich durch eine sehr gute fungizide Wirkung aus. Mit ihrer Hilfe lassen sich bereits in das pflanzliche Gewebe eingedrungene pilzliche Krankheitserreger kurativ bekämpfen. Dies ist besonders wichtig und vorteilhaft bei solchen Pilzkrankheiten, die nach eingetretener Infektion mit den sonst üblichen Fungiziden nicht mehr bekämpft werden können. Das Wirkungsspektrum der beanspruchten Verbindungen erfaßt neben Phytophthora infestans, Fusicladium dendriticum, Plasmopara viticola, Piricularia oryzae und Puccinia triticina vor allem Echte Mehltauarten im Obst-, Gemüse-, Getreide- und Zierpflanzenanbau. Besonders hervorzuheben ist die ausgezeichnete Wirkung der Verbindungen gegen Benzimidazolcarbamat-resistente Mehltauarten.

Für die Anwendung im Pflanzenschutz können die Verbindungen der Formel I in üblicher Weise, z.B. als Stäube, Spritzpulver, Beizmittel, Dispersionen, Lösungen oder Emulsionskonzentrate formuliert werden. Der Gehalt an Wirkstoff der Formel I liegt in den fungiziden Mitteln im allgemeinen zwischen 2 bis 95 Gew.-%, vorzugsweise bei 10 bis 90 Gew.-%. Daneben enthalten die genannten Wirkstoff-Formulierungen gegebenenfalls die jeweils üblichen Haft-, Netz-, Dispergier-, Emulgier-, Penetrations-, Lösungsmittel-, Füll- und Trägerstoffe.

Die beanspruchten Verbindungen der Formel I eignen sich auch für den Einsatz im technischen Bereich, beispielsweise in Holzschutzmitteln, auf dem Anstrichfarbensektor oder als Konservierungsmittel, z.B. in Kühlschmiermitteln für die Metallbearbeitung.

Die Erfindung wird durch folgende Beispiele näher erläutert:

## A. Herstellungsbeispiele

### Beispiel 1

Diäthyl-2-chlor-2-/(phenyl)-(1,2,4-triazol-1-yl)-methyl/-malonat

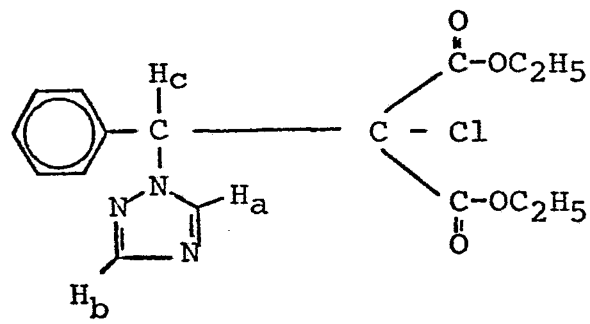

a) Ein Gemisch aus 269,5 g (0,85 Mol) Diäthyl-2-/(phenyl)-(1,2,4-triazol-1-yl)-methyl/malonat, 94,4 g (0,283 Mol) Hexachlormelamin und 600 ml Tetrachlorkohlenstoff wird 9 Stunden bei Rückflußtemperatur (ca. 80°C) gerührt. Dabei gehen zunächst die Ausgangskomponenten in Lösung und nach ca. 1,5 Stunden beginnt ein Feststoff, der hauptsächlich aus Melaminbestandteilen besteht, auszufallen. Man läßt das Reaktionsgemisch abkühlen, filtriert und engt das Filtrat unter Vakuum ein.

Man erhält ein gelbliches Öl, das mit einem Gemisch aus 300 ml Diisopropyläther und 20 ml Chloroform versetzt und 3,5 Stunden bei Normaltemperatur gerührt wird. Dabei fällt weiterer Feststoff aus, der, wie oben beschrieben, von der Lösung des Reaktionsproduktes abgetrennt wird. Die Lösungsmittel werden unter Vakuum von der Lösung abdestilliert und man erhält 292,2 g (= 97,8 % der Theorie) Diäthyl-2-chlor-2/(phenyl)-(1,2,4-triazol-1-yl)methyl/malonat als gelbes Öl, das gemäß $^1$H-NMR-Analyse frei ist von nichtchloriertem Ausgangsprodukt.

Analyse:

$^1$H-NMR ($CDCl_3$, $\delta$ in ppm, J in Hz)

$\delta$ = 1,11 (t, $^3J_{H,H}$ = 7,3 Hz, $-O-CH_2-\underline{CH_3}$ )
$\delta$ = 1,23 (t, $^3J_{H,H}$ = 7,3 Hz, $-O-CH_2-\underline{CH_3}$ )
$\delta$ = 4,15 (q, $^3J_{H,H}$ = 7,3 Hz, $-O-\underline{CH_2}-CH_3$ )
$\delta$ = 4,27 (q, $^3J_{H,H}$ = 7,3 Hz, $-O-\underline{CH_2}-CH_3$ )
$\delta$ = 6,63 (s, $H_c$)
$\delta$ = 7,2-7,75 (m, Aromaten H)
$\delta$ = 7,94 (s, $H_b$)
$\delta$ = 8,23 (s, $H_a$)

b) Ein Gemisch aus 15,85 g (0,05 Mol) Diäthyl-2[(phenyl)-(1,2,4-triazol-1-yl)methyl]malonat, 13,35 g (0,1 Mol) N-Chlorsuccinimid und 50 ml Tetrachlorkohlenstoff wird 13 Stunden bei Rückflußtemperatur (ca. 80°C) gerührt. Man läßt anschließend auf Raumtemperatur abkühlen, saugt vom ausgefallenen Feststoff ab, engt das Filtrat unter Wasserstrahlvakuum ein und erhält als gewünschtes Reaktionsprodukt ein gelbliches Öl, das unter Hochvakuum bei ca. 30°C von letzten Lösungsmittelresten befreit wird.
Ausbeute: 17,48 g (= 99,5 % der Theorie) gelbliches Öl, das gemäß $^1$H-NMR ($CDCl_3$)-Analyse identisch ist mit dem Produkt aus Beispiel 1a).

c) Ein Gemisch aus 12,68 g (0,04 Mol) Diäthyl-2[(phenyl)-(1,2,4-triazol-1-yl)methyl]malonat, 2,78 g (0,01 Mol) Hexachlormelamin und 40 ml tert.- Butanol wird 9 Stunden bei Rückflußtemperatur (ca. 82°C) gerührt. Man trennt die Lösung vom ausgefallenen Feststoff durch Filtrieren ab, engt das Filtrat unter Vakuum ein und erhält 13,51 g (= 96,1 % der Theorie) Diäthyl-2-chlor-2-[(phenyl)-1,2,4-triazol-1-yl)methyl]malonat als gelbes Öl.

0021377

Das $^1H$-NMR ($CDCl_3$)-Spektrum des Produktes ist identisch mit dem $^1H$-NMR ($CDCl_3$)-Spektrum des nach Beispiel 1a) erhaltenen Produktes.

Beispiel 2

Äthyl-2-chlor-2/̅(4-chlorphenyl)-(1,2,4-triazol-1-yl)-methyl̲7-3-oxo-butanoat

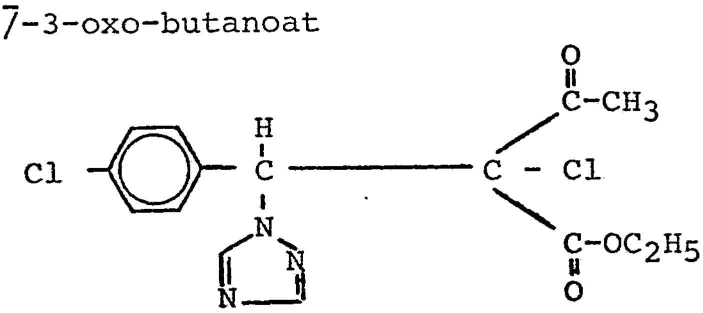

Ein Gemisch aus 17,68 g (0,055 Mol) Äthyl-2/(4-chlorphenyl)-(1,2,4-triazol-1-yl)-methyl/-3-oxo-butanoat, 4,88 g (0,0147 Mol) Hexachlormelamin und 40 ml Tetrachlorkohlenstoff wird unter Rühren 8 Stunden auf Rückflußtemperatur (ca. 80°C) erhitzt. Man läßt das Reaktionsgemisch abkühlen, filtriert die Lösung und destilliert das Lösungsmittel unter Vakuum bei ca. 25°C ab. Nach Trocknen des Lösungsrückstandes unter Hochvakuum verbleiben 18,95 g (= 96,8 % der Theorie) Äthyl-2-chlor-2/(4-chlorphenyl)-(1,2,4-triazol-1-yl)-methyl/-3-oxo-butanoat als gelbes Öl.

Analyse:

| | | | | |
|---|---|---|---|---|
| ber.: | C 50,58 %; | H 4,24 %; | N 11,8 %; | Cl 19,9 % |
| gef.: | C 50,2 %; | H 4,2 %; | N 11,6 %; | Cl 20,0 % |

Beispiele 3 - 20

In der Tabelle 1 sind die Reste $R^1$, $R^2$, $R^3$ und X in Formel I der nach den Beispielen 3 bis 20 aus den entsprechenden Verbindungen der Formel II durch Halogenierung hergestellten Verbindungen der Formel I sowie deren physikalisch-chemische Kenndaten, wie Brechungsindex (n) Schmelzpunkt (Fp.) bzw. Elementaranalyse (C,H,N) aufgeführt. Im einzelnen wird bei deren Herstellung wie folgt verfahren:

Die Beispiele 8, 12, 13, 15, 16, 19 und 20 werden analog zu Beispiel 1a) durchgeführt.

Die Beispiele 5, 6 und 11 werden analog zu Beispiel 1b) durchgeführt, jedoch unter Verwendung von äquivalenten Mengen N-Bromsuccinimid anstelle von N-Chlorsuccinimid.

Die Beispiele 3, 4, 7, 9, 10, 14, 17 und 18 werden analog zu Beispiel 2 durchgeführt.

Tabelle 1

Formel I: 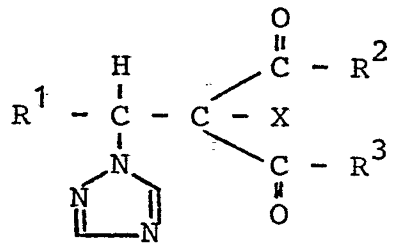 (I)

| Beispiel | $R^1$ | $R^2$ | $R^3$ | X | Kenndaten |
|---|---|---|---|---|---|
| 3 | 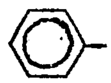 | $CH_3-$ | $CH_3O-$ | Cl | $n_D^{30}$ = 1,5401 |
| 4 | 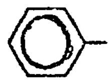 | $CH_3-$ | $C_2H_5O-$ | Cl | $n_D^{22}$ = 1,5401 |
| 5 | 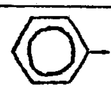 | $CH_3-$ | $C_2H_5O-$ | Br | |
| 6 | 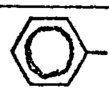 | $C_2H_5O-$ | $C_2H_5O-$ | Br | $n_D^{30}$ = 1,5360 |
| 7 | 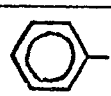 | $n-C_3H_7-$ | $C_2H_5O-$ | Cl | $n_D^{30}$ = 1,5276 |
| 8 | 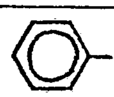 | $n-C_3H_7O-$ | $n-C_3H_7O-$ | Cl | $n_D^{30}$ = 1,5185 |
| 9 | 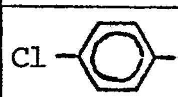 | $CH_3-$ | $CH_3-$ | Cl | $n_D^{30}$ = 1,5594 |
| 10 | 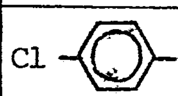 | $CH_3-$ | $CH_3O-$ | Cl | $n_D^{30}$ = 1,5558 |
| 11 | 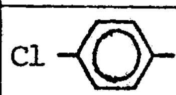 | $CH_3-$ | $C_2H_5O-$ | Br | |
| 12 | 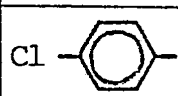 | $CH_3O-$ | $CH_3O-$ | Cl | |
| 13 | 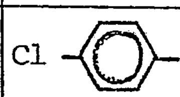 | $C_2H_5O-$ | $C_2H_5O-$ | Cl | $n_D^{30}$ = 1,5308 |
| 14 | 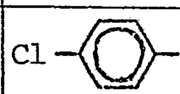 | $n-C_3H_7-$ | $C_2H_5O-$ | Cl | $n_D^{30}$ = 1,5338 |
| 15 | 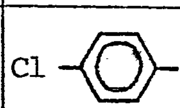 | $n-C_3H_7O-$ | $n-C_3H_7O-$ | Cl | ber. C 52,19 %; H 5,1%; N 10,14% gef. C 51,8 %; H 4,8 %; N 10,1 % |

| Beispiel | $R^1$ | $R^2$ | $R^3$ | X | Kenndaten |
|---|---|---|---|---|---|
| 16 | Cl— 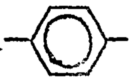 | iso-$C_3H_7O$- | iso-$C_3H_7O$- | Cl | $n_D^{30}$= 1,5280 |
| 17 | $CH_3$— 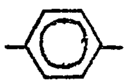 | $CH_3$- | $CH_3$- | Cl | $n_D^{30}$= 1,5485 |
| 18 | $CH_3$— 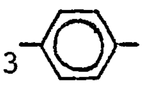 | $CH_3$- | $C_2H_5O$- | Cl | $n_D^{30}$= 1,5343 |
| 19 | 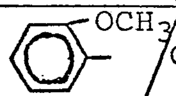 | $C_2H_5O$- | $C_2H_5O$- | Cl | Fp.= 91-94°C |
| 20 | n-$C_3H_7$- | $CH_3O$- | $CH_3O$- | Cl | $n_D^{30}$= 1,4798 |

B. Formulierungsbeispiele

Beispiel A

Ein Stäubemittel wird erhalten, indem man
10 Gewichtsteile Wirkstoff
und 90 Gewichtsteile Talkum als Inertstoff
mischt und in einer Schlagmühle zerkleinert.

Beispiel B

Ein in Wasser leicht dispergierbares, benetzbares Pulver wird erhalten, indem man
25 Gewichtsteile Wirkstoff
64 Gewichtsteile kaolinhaltigen Quarz als Inertstoff
10 Gewichtsteile ligninsulfonsaures Natrium
und 1 Gewichtsteil oleoylmethyltaurinsaures Natrium als Netz- und Dispergiermittel
mischt und in einer Stiftmühle mahlt.

Beispiel C

Ein in Wasser leicht dispergierbares Dispersionskonzentrat wird erhalten, indem man
20 Gewichtsteile Wirkstoff mit
6 Gewichtsteilen Nonylphenolpolyglykoläther (10AeO)
3 Gewichtsteilen Isotridecanolpolyglykoläther (8AeO)
und 71 Gewichtsteilen paraffinischem Mineralöl (Siedebereich 255 bis > 377°C/Normaldruck)
mischt und in einer Reibkugelmühle auf eine Feinheit von unter 5 Mikron vermahlt.

Beispiel D

Ein emulgierbares Konzentrat wird erhalten aus
15 Gewichtsteilen Wirkstoff
75 Gewichtsteilen Cyclohexanon als Lösungsmittel
und 10 Gewichtsteilen oxäthyliertes Nonylphenol (10 AeO) als Emulgator.

0021377

## C. Biologische Beispiele

In den folgenden biologischen Beispielen stehen die Buchstaben A, B, C, D und E für die nachstehend genannten handelsüblichen Vergleichsmittel:

A: Methyl-1-(butylcarbamoyl)-2-benzimidazolcarbamat (Benomyl)

B: N-Tridecyl-2,6-dimethyl-morpholin (Tridemorph)

C: 2,4-Dinitro-6-sek.butyl-phenyl-3,3-dimethyl-acrylat (Binapacryl)

D: Mergal AF (Kombination von Chloracetamid mit einer quarternären Ammoniumverbindung und einem Fluorid).

E: Mergal 588 (Kombination von Carbendazim (BCM) mit einem Dithiocarbamat)

### Beispiel I

Weizenpflanzen werden im 3-Blattstadium mit Konidien des Weizenmehltaus (Erysiphe graminis) stark inokuliert und in einem Gewächshaus bei 20°C und einer relativen Luftfeuchte von 90 - 95 % aufgestellt. 3 Tage nach Inokulation werden die Pflanzen mit den in Tabelle I aufgeführten Verbindungen in den Wirkstoffkonzentrationen von 500, 250,125, 60, 30 und 15 mg/Liter Spritzbrühe tropfnaß gespritzt. Als Vergleich wird das Vergleichsmittel B eingesetzt. Nach einer Inkubationszeit von 10 Tagen werden die Pflanzen auf Befall mit Weizenmehltau untersucht. Der Befallsgrad wird ausgedrückt in % befallener Blattfläche, bezogen auf unbehandelte, infizierte Kontrollpflanzen (= 100 % Befall). Das Ergebnis ist in der Tabelle I zusammengefaßt.

Tabelle I

| Verbindung gemäß Beispiel | mit Weizenmehltau befallene Blattfläche in % bei mg Wirkstoff/Liter Spritzbrühe | | | | | |
|---|---|---|---|---|---|---|
| | 500 | 250 | 125 | 60 | 30 | 15 |
| 3 | 0 | 0 | 0 | 0 | 0 | 0 - 3 |
| 1 | 0 | 0 | 0 | 0 | 0 | 0 |
| 13 | 0 | 0 | 0 | 0 | 0 | 0 |
| 10 | 0 | 0 | 0 | 0 | 0 - 3 | 3 - 5 |
| 18 | 0 | 0 | 0 | 0 | 0 - 3 | 3 - 5 |
| 14 | 0 | 0 | 0 | 0 | 0 | 0 |
| 7 | 0 | 0 | 0 | 0 | 0 | 0 - 3 |
| 2 | 0 | 0 | 0 | 0 | 0 - 3 | 3 |
| 12 | 0 | 0 | 0 | 0 | 0 - 3 | 3 |
| 11 | 0 | 0 | 0 | 0 - 3 | 3 | 5 |
| 15 | 0 | 0 | 0 | 0 | 0 | 0 |
| 8 | 0 | 0 | 0 | 0 | 0 | 0 |
| 16 | 0 | 0 | 0 | 0 | 0 - 3 | 3 |
| 6 | 0 | 0 | 0 | 0 | 0 - 3 | 3 |
| Vergleichsmittel B | 3 phytotoxisch | 5 phytotoxisch | 10 | 15 | 25 | 35 |
| unbeh., inf. Pflanzen | 100 | | | | | |

Beispiel II

Gurkenpflanzen (Sorte Delikateß) werden im 2-Blattstadium mit einer Konidiensuspension von Gurkenmehltau (Erysiphe cichora-cearum) stark inokuliert. Nach einer Antrocknungszeit der Sporensuspension von 30 Minuten werden die Pflanzen in einem Gewächshaus bei 22°C und 90 % relativer Luftfeuchte aufgestellt. 3 Tage nach Infektion werden die Pflanzen mit den in Tablle II genannten Verbindungen und Wirkstoffkonzentrationen tropfnaß gespritzt. Als Vergleich wird das Vergleichsmittel A eingesetzt. Nach 10 Tagen erfolgt die Bonitur. Der

Befallsgrad wird ausgedrückt in % befallener Blattfläche, bezogen auf unbehandelte, infizierte Kontrollpflanzen (= 100 % Befall). Das Ergebnis ist in der Tabelle II zusammengefaßt.

Tabelle II

| Verbindung gemäß Beispiel | mit Gurkenmehltau befallene Blattfläche in % bei mg Wirkstoff/Liter Spritzbrühe | | | | | |
|---|---|---|---|---|---|---|
| | 500 | 250 | 125 | 60 | 30 | 15 |
| 3 | 0 | 0 | 0 | 0 | 0 - 3 | 3 |
| 1 | 0 | 0 | 0 | 0 | 0 | 0 |
| 13 | 0 | 0 | 0 | 0 | 0 | 0 |
| 10 | 0 | 0 | 0 | 0 | 0 | 0 - 3 |
| 18 | 0 | 0 | 0 | 0 | 0 | 0 |
| 14 | 0 | 0 | 0 | 0 | 0 | 0 |
| 7 | 0 | 0 | 0 | 0 | 0 | 0 |
| 2 | 0 | 0 | 0 | 0 | 0 | 0 |
| 12 | 0 | 0 | 0 | 0 | 0 - 3 | 3 |
| 15 | 0 | 0 | 0 | 0 | 0 | 0 |
| 8 | 0 | 0 | 0 | 0 | 0 | 0 |
| 16 | 0 | 0 | 0 | 0 | 0 - 3 | 3 |
| 6 | 0 | 0 | 0 | 0 | 0 - 3 | 3 |
| Vergleichsmittel A | 3 | 5 | 10 | 15 | 25 | 60 |
| unbeh., inf. Pflanzen | 100 | | | | | |

Beispiel III

Gurkenpflanzen (Sorte Delikateß) werden im 2-Blattstadium mit einer Konidiensuspension eines Benomyl-resistenten Gurkenmehltaustammes (Erysiphe cichoracearum) stark inokuliert. Nach einer Antrocknungszeit der Sporensuspension von 30 Minuten werden die Pflanzen in einem Gewächshaus bei 22°C und 90 % relativer Luftfeuchte aufgestellt. 3 Tage nach Infektion werden die Pflanzen mit den in Tabelle III genannten Verbindungen und Wirkstoffkonzentrationen tropfnaß gespritzt. Als Vergleich wird das Vergleichsmittel A eingesetzt. Nach 10 Tagen erfolgt die Bonitur. Der Befallsgrad wird ausgedrückt in % befallener Blattfläche, bezogen auf unbehandelte, infizierte Kontrollpflanzen (= 100 % Befall). Das Ergebnis ist in der Tabelle III zusammengefaßt.

Tabelle III

| Verbindung gemäß Beispiel | mit Gurkenmehltau (Benomyl-resistenter Stamm) befallene Blattfläche in % bei mg Wirkstoff/ Liter Spritzbrühe | | | | | |
|---|---|---|---|---|---|---|
| | 500 | 250 | 125 | 60 | 30 | 15 |
| 3 | 0 | 0 | 0 | 0 | 0 - 3 | 3 |
| 1 | 0 | 0 | 0 | 0 | 0 | 0 |
| 13 | 0 | 0 | 0 | 0 | 0 | 0 |
| 10 | 0 | 0 | 0 | 0 | 0 | 0 - 3 |
| 18 | 0 | 0 | 0 | 0 | 0 | 0 |
| 14 | 0 | 0 | 0 | 0 | 0 | 0 |
| 7 | 0 | 0 | 0 | 0 | 0 | 0 - 3 |
| 2 | 0 | 0 | 0 | 0 | 0 | 0 |
| 12 | 0 | 0 | 0 | 0 | 0 - 3 | 3 |
| 15 | 0 | 0 | 0 | 0 | 0 | 0 |
| 8 | 0 | 0 | 0 | 0 | 0 | 0 |
| 16 | 0 | 0 | 0 | 0 | 0 - 3 | 3 |
| 6 | 0 | 0 | 0 | 0 | 0 | 0 - 3 |
| Vergleichsmittel A | 35 | 60 | 100 | 100 | 100 | 100 |
| unbeh., inf. Pflanzen | 100 | | | | | |

Beispiel IV

Gerstenpflanzen werden im 3-Blattstadium mit Konidien des Gerstenmehltaus (Erysiphe graminis sp. hordei) stark inokuliert und in einem Gewächshaus bei 20°C und einer relativen Luftfeuchte von 90 - 95 % aufgestellt. 3 Tage nach Inokulation werden die Pflanzen mit den in Tabelle IV aufgeführten Verbindungen in den Wirkstoffkonzentrationen von 500, 250, 125, 60, 30 und 15 mg/Liter Spritzbrühe tropfnaß gespritzt. Als Vergleich wird das Vergleichsmittel B eingesetzt. Nach einer Inkubationszeit von 10 Tagen werden die Pflanzen auf Befall mit Gerstenmehltau untersucht. Der Befallsgrad wird ausgedrückt in % befallener Blattfläche, bezogen auf unbehandelte, infizierte Kontrollpflanzen (= 100 % Befall). Das Ergebnis ist in der Tabelle IV zusammengefaßt.

Tabelle IV

| Verbindung gemäß Beispiel | mit Gerstenmehltau befallene Blattfläche in % bei mg Wirkstoff/Liter Spritzbrühe | | | | | |
|---|---|---|---|---|---|---|
| | 500 | 250 | 125 | 60 | 30 | 15 |
| 3 | 0 | 0 | 0 | 0 - 3 | 3 | 3 - 5 |
| 1 | 0 | 0 | 0 | 0 | 0 | 0 |
| 13 | 0 | 0 | 0 | 0 | 0 | 0 |
| 10 | 0 | 0 | 0 | 0 | 0 - 3 | 3 |
| 18 | 0 | 0 | 0 | 0 | 0 | 0 - 3 |
| 14 | 0 | 0 | 0 | 0 | 0 | 0 |
| 7 | 0 | 0 | 0 | 0 | 0 | 0 |
| 2 | 0 | 0 | 0 | 0 | 0 | 0 - 3 |
| 12 | 0 | 0 | 0 | 0 | 0 | 0 - 3 |
| 15 | 0 | 0 | 0 | 0 | 0 | 0 - 3 |
| 8 | 0 | 0 | 0 | 0 | 0 | 0 |
| 19 | 0 | 0 | 0 | 0 | 0 | 0 |
| 16 | 0 | 0 | 0 | 0 | 0 - 3 | 3 |

0021377

| Verbindung gemäß Beispiel | mit Gerstenmehltau befallene Blattfläche in % bei mg Wirkstoff/Liter Spritzbrühe | | | | | |
|---|---|---|---|---|---|---|
| | 500 | 250 | 125 | 60 | 30 | 15 |
| 6 | 0 | 0 | 0 | 0 | 0 | 0 - 3 |
| Vergleichs-mittel B | 3 | 3 - 5 | 5 | 5 - 10 | 15 | 25 |
| unbeh., inf. Pflanzen | 100 | | | | | |

Beispiel V

Jeweils 0,02 ml einer Sporensuspension von Ulocladium consortiale, Aspergillus niger und Penicillium funiculosum werden jeweils in verschiedene Petrischalen auf Nährboden (Biomalz-Agar für Pilze) tropfenförmig aufgebracht; dem Agar sind zuvor im flüssigen Zustand die erfindungsgemäßen Verbindungen der angegebenen Beispiele in den in Tabelle V angegebenen Konzentrationen als Wirkstoff zugesetzt worden. 6 Tage nach der Beimpfung der Platten wird der Durchmesser der Pilzkolonien auf dem Agar ausgemessen und die durch die Präparate hervorgerufene Wachstumshemmung, ausgedrückt in %, bezogen auf die Kontrolle (= beimpfter Agar ohne Wirkstoff-Zusatz= 0 % Hemmung), ermittelt. Das Ergebnis ist in der Tabelle V zusammengefaßt.

Tabelle V

| Verbindung gemäß Beispiel | Sporenart | Wachstumshemmung in % bei mg Wirkstoff/Ltr. Agar | | | |
|---|---|---|---|---|---|
| | | 1000 | 500 | 100 | 50 |
| 1 | U. consortiale | 100 | 100 | 100 | 100 |
| | A. niger | 100 | 100 | 100 | 100 |
| | P. funiculosum | 100 | 100 | 100 | 100 |

| Verbindung gemäß Beispiel | Sporenart | Wachstumshemmung in % bei mg Wirkstoff/Ltr. Agar | | | |
|---|---|---|---|---|---|
| | | 1000 | 500 | 100 | 50 |
| 11 | A. niger | 100 | 100 | 80 | 50 |
| | P. funiculosum | 100 | 100 | 100 | 50 |
| 17 | U. consortiale | 100 | 100 | 100 | 100 |
| | A. niger | 100 | 100 | 100 | 50 |
| | P. funiculosum | 100 | 100 | 100 | 50 |
| Vergleichsmittel E | U. consortiale | 100 | 100 | 50 | 0 |
| | A. niger | 100 | 100 | 100 | 0 |
| | P. funiculosum | 100 | 100 | 100 | 0 |

Patentansprüche:

1. Verbindungen der allgemeinen Formel I,

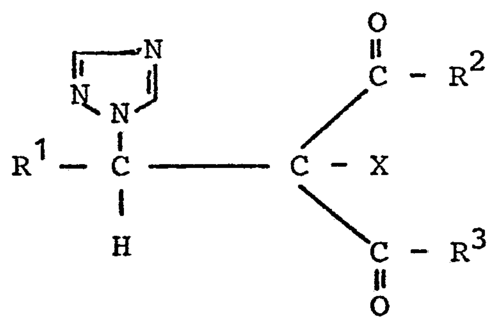

(I)

worin

$R^1$ $(C_1-C_{12})$-Alkyl, Cycloalkyl mit vorzugsweise 5 oder 6 C-Atomen, Cycloalkenyl mit vorzugsweise 5 oder 6 C-Atomen, Phenyl, substituiertes Phenyl, insbesondere ein- bis dreifach durch Alkyl, vorzugsweise $(C_1-C_{12})$-Alkyl, Halogen, $(C_1-C_5)$-Alkoxy, Hydroxy, Nitro oder Dialkylamino mit vorzugsweise $(C_1-C_6)$-Alkylgruppen substituiertes Phenyl, weiterhin Furanyl, Thienyl oder Pyridyl, und

$R^2, R^3$ $(C_1-C_{12})$-Alkyl, Cycloalkyl mit vorzugsweise 5 oder 6 C-Atomen, Phenyl, substituiertes Phenyl, insbesondere ein- bis dreifach durch Alkyl, vorzugsweise $(C_1-C_{12})$-Alkyl, Halogen, $(C_1-C_5)$-Alkoxy oder Hydroxy substituiertes Phenyl, oder $(C_1-C_{12})$-Alkoxy, $(C_5-C_6)$Cycloalkoxy oder Benzyloxy, und

X Chlor oder Brom, vorzugsweise Chlor bedeuten.

2. Verfahren zur Herstellung von Verbindungen der Formel I gemäß Anspruch 1, dadurch gekennzeichnet, daß man Verbindungen der allgemeinen Formel II,

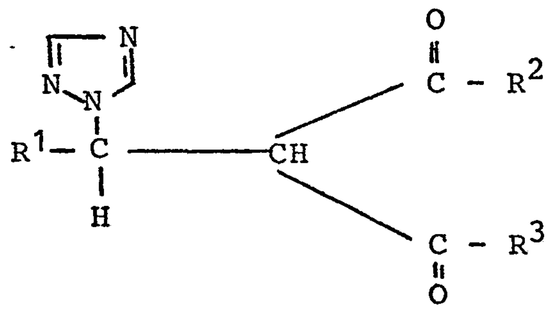

(II)

worin $R^1$, $R^2$, $R^3$ die Bedeutungen wie in Formel I haben, mit einem Halogenierungsmittel umsetzt.

3. Verfahren nach Anspruch 2, dadurch gekennzeichnet, daß man die Umsetzung in Gegenwart eines Lösungsmittels durchführt.

4. Verfahren nach Ansprüchen 2 und 3, dadurch gekennzeichnet, daß man als Halogenierungsmittel N-Halogenamine einsetzt.

5. Schädlingsbekämpfungsmittel, gekennzeichnet durch einen Gehalt an einer Verbindung der Formel I gemäß Anspruch 1 als Wirkstoff.

6. Schädlingsbekämpfungsmittel, gekennzeichnet durch einen Gehalt von 2 bis 95 Gew.-%, vorzugsweise 10 bis 90 Gew.-% an einer Verbindung der Formel I gemäß Anspruch 1 als Wirkstoff sowie üblichen Formulierungshilfsmitteln.

7. Verwendung von Verbindungen der Formel I gemäß Ansprüchen 1, 5 und 6 zur Schädlingsbekämpfung im Pflanzenschutz.

8. Verfahren zur Bekämpfung von Schadpilzen, dadurch gekennzeichnet, daß man die von ihnen befallenen Flächen, Pflanzen oder Substrate mit einer fungizid wirksamen Menge von Wirkstoffen der Formel I gemäß Ansprüchen 1,5 und 6 in Kontakt bringt.

Patentansprüche für Österreich

1. Verfahren zur Herstellung von Verbindungen der allgemeinen Formel I,

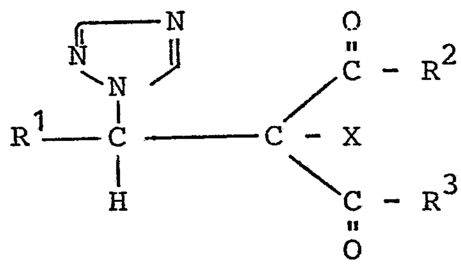

(I)

worin

$R^1$ $(C_1-C_{12})$-Alkyl, Cycloalkyl mit vorzugsweise 5 oder 6 C-Atomen, Cycloalkenyl mit vorzugsweise 5 oder 6 C-Atomen, Phenyl, substituiertes Phenyl, insbesondere ein- bis dreifach durch Alkyl, vorzugsweise $(C_1-C_{12})$-Alkyl, Halogen, $(C_1-C_5)$-Alkoxy, Hydroxy, Nitro oder Dialkylamino mit vorzugsweise $(C_1-C_6)$-Alkylgruppen substituiertes Phenyl, weiterhin Furanyl, Thienyl oder Pyridyl, und

$R^2,R^3$ $(C_1-C_{12})$-Alkyl, Cycloalkyl mit vorzugsweise 5 oder 6 C-Atomen, Phenyl, substituiertes Phenyl, insbesondere ein- bis dreifach durch Alkyl, vorzugsweise $(C_1-C_{12})$-Alkyl, Halogen, $(C_1-C_5)$-Alkoxy oder Hydroxy substituiertes Phenyl, oder $(C_1-C_{12})$-Alkoxy, $(C_5-C_6)$ Cycloalkoxy oder Benzyloxy, und

X Chlor oder Brom, vorzugsweise Chlor, bedeuten, dadurch gekennzeichnet, daß man Verbindungen der allgemeinen Formel II,

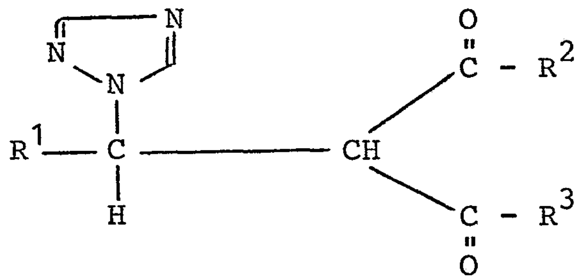

(II)

worin $R^1$, $R^2$, $R^3$ die Bedeutungen wie in Formel I haben, mit einem Halogenierungsmittel umsetzt.

2. Verfahren nach Anspruch 1, dadurch gekennzeichnet, daß man die Umsetzung in Gegenwart eines Lösungsmittels durchführt.

3. Verfahren nach Ansprüchen 1 und 2, dadurch gekennzeichnet, daß man als Halogenierungsmittel N-Halogenamine einsetzt.

4. Schädlingsbekämpfungsmittel, gekennzeichnet durch einen Gehalt an einer Verbindung der Formel I gemäß Anspruch 1 als Wirkstoff.

5. Schädlingsbekämpfungsmittel, gekennzeichnet durch einen Gehalt von 2 bis 95 Gew.-%, vorzugsweise 10 bis 90 Gew.-% an einer Verbindung der Formel I gemäß Anspruch 1 als Wirkstoff sowie üblichen Formulierungshilfsmitteln.

6. Verwendung von Verbindungen der Formel I gemäß Ansprüchen 1, 4 und 5 zur Schädlingsbekämpfung im Pflanzenschutz.

7. Verfahren zur Bekämpfung von Schadpilzen, dadurch gekennzeichnet, daß man die von ihnen befallenen Flächen, Pflanzen oder Substrate mit einer fungizid wirksamen Menge von Wirkstoffen der Formel I gemäß Ansprüchen 1, 4 und 5 in Kontakt bringt.

0021377
Europäisches Patentamt

# EUROPÄISCHER RECHERCHENBERICHT

Nummer der Anmeldung
EP 80 10 3490

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| P | DE - A - 2 831 235 (BASF)<br>* Patentansprüche Seiten 1,2,3 *<br>---- | 1,5-8 | C 07 D 249/08<br>403/06<br>405/06<br>407/06<br>A 01 N 43/64 |

RECHERCHIERTE SACHGEBIETE (Int. Cl.³)

C 07 D 249/08

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 25-09-1980 | CREMERS |